# EUROPEAN PATENT SPECIFICATION

(11) **EP 3 520 682 B1**
(45) Date of publication and mention of the grant of the patent: **12.02.2020**
(21) Application number: 17800560.9
(22) Date of filing: 22.09.2017
(51) Int. Cl.: A61B 3/16

(54) **APPLANATION TONOMETER**
APPLANATIONSTONOMETER
TONOMÈTRE À APLANATION

(30) Priority: 03.10.2016 ES 201631280
(43) Date of publication of application: 07.08.2019
(73) Proprietor: Iglesias Alvarez, Maria, 32004 Ourense (ES)
(72) Inventor: Iglesias Alvarez, Maria, 32004 Ourense (ES)
(74) Representative: Ungria López, Javier
(86) International application number: PCT/ES2017/070626
(87) International publication number: WO 2018/065646

(56) References cited:
- WO-A1-2015/074094
- WO-A1-2016/167827
- NL-A- 7 408 214
- SU-A1- 1 731 158
- US-A- 3 070 997
- US-A1- 2014 073 897

## Description

The present invention is an ophthalmological device that measures intraocular pressure, named applanation tonometer. It allows the measurement of intraocular pressure in myopic patients undergoing laser assisted in situ keratomileusis (LASIK) or photorefractive keratectomy (PRK) refractive surgery.

LASIK is a refractive surgery for the correction of myopia, hypermetropia and astigmatism. It is performed by an ophthalmologist who uses a low-power laser to permanently alter the shape of the cornea, in order to improve vision and reduce the need for the operated person to wear glasses or contact lenses.

PRK is a surgical procedure that uses laser in a similar way as LASIK to treat refractive defects of the eye, such as myopia, hypermetropia and astigmatism.

A convex surface is curved outwardly, having its protruding portion directed towards the observer. It is the concept opposite to concavity.

The initials IOP correspond to intraocular pressure. It is the pressure exerted by the intraocular fluid against the wall of the eye, which is necessary for this organ to remain distended.

It is of great importance to find a reliable and reproducible IOP measurement system, since myopia is an important risk factor for developing glaucoma. Myopic LASIK and PRK operated patients cannot be measured reliable with the actual applanation tonometers.

The Goldman tonometer (from now on referred as GT) is a reference device in ophthalmology for IOP measurement.

It has its origins in patent documents GB862920 and US2968941 which describe the first applanation tonometers.

In this tonometer, the objective measurement of IOP is based on the force required to flatten the cornea according to the Imbert-Fick law. This law postulates that the pressure inside a sphere (ideal, with dry and thin walls) is equal to the force necessary to flatten the surface divided by the area of flattening. However in the human eye we must take into account that the cornea is rigid (offers resistance to flattening) and the capillary attraction of the meniscus attracts the cornea. The GT is able to cancel these two forces when the applanation area has a diameter of 3.06 mm.

A GT is composed by a transparent, optical bi-prism with a flat apex that contacts with the anterior surface of the cornea in an area of 3.06 mm of diameter (contact area of 7.354 mm2). The optic bi-prism is divided in two parts, to allow the ophthalmologist to determine when this specific contact area (of 3.06 mm) has been reached, by observing inside the bi-prism two semi-circular images coincide in a curved line with the form of an "S".

Nevertheless, GT is only reliable when the central corneal thickness (from now on referred as CCT) is approximately 520 µm, so its validity has been questioned in corneas above and below this value. Also, when GT is used on a thinner cornea - like in patients operated on with LASIK or PRK- produces a greater applanation area than normal 3.06 mm, indicating much lower pressure than the actual one.

In parallel there are also applanation tonometers with a flexible convex diaphragm shape, as described in the patent document SU1731158. When it makes contact with the eye, the diaphragm deforms, which makes the inner membrane indicating the pressure by the indicator. This tonometer lacks optical bi-prism.

US20140073897 discloses an applanation tonometer similar to GT, with a convex-concave contact surface, being the corneal contacting surface concave. The concave apex is adapted to the shape of the cornea. The part with convex shape is a consequence of the manufacturing process.

The aim of our invention is to make known a new device that solves the problem posed to measure the IOP in cases of patients undergoing myopic laser refractive surgery.

To this end, the present invention provides an applanation tonometer to measure IOP comprising a transparent optical bi-prism with a distal apex intended to make contact with the anterior surface of the cornea. This apex is curved and convex shape, as seen from the outside of the bi-prism.

Preferably, the apex of the optical bi-prism has a curvature with a mean surface radius in the range of 11.5 to 14 millimetres.

Apart from the convex apex, the invention also provides another difference with respect to GT and other applanation tonometers: the tip of the cone has a 7.06 mm diameter, unlike that of GT measuring approximately 7.02 mm. This would have no relevant differences in the tonometer weight.

As it has been described, GT is only reliable in corneas with an average CCT. In myopic operated cases, in which the corneas are thinner and flattened, the convexity of the bi-prism of this new invention may compensate the absence of corneal tissue due to the surgery, an so on, it can compensate for the variation of IOP measurement compared GT. Therefore, when IOP is measured with a convex-shaped apex tonometer in a patient operated on LASIK or PRK, the IOP obtained is very close to that obtained prior to surgery with a flat apex (TG).

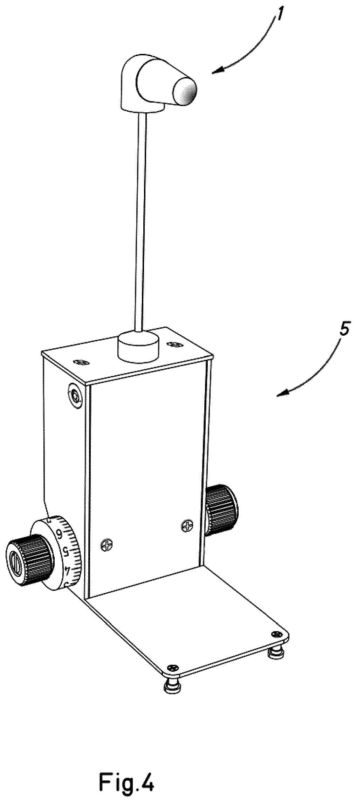

Another advantage of the present invention is that, as a GT modification, it can be inserted into both, the slit lamp of conventional use (Figure 4) or in Perkins tonometer, both used in daily clinical practice with a minimum cost with respect to other different tonometric models.

In addition, the procedure of measuring the IOP with this new device is the same as with the GT. To this end, the ophthalmologist must exchange GT with the convex-apex bi-prism of the present invention, in the slit lamp or in Perkins tonometer, in order to measure the IOP in the subgroup of patients mentioned above.

The present device can be manufactured -with the appropriate materials- either in disposable or reusable for sterilization prototypes.

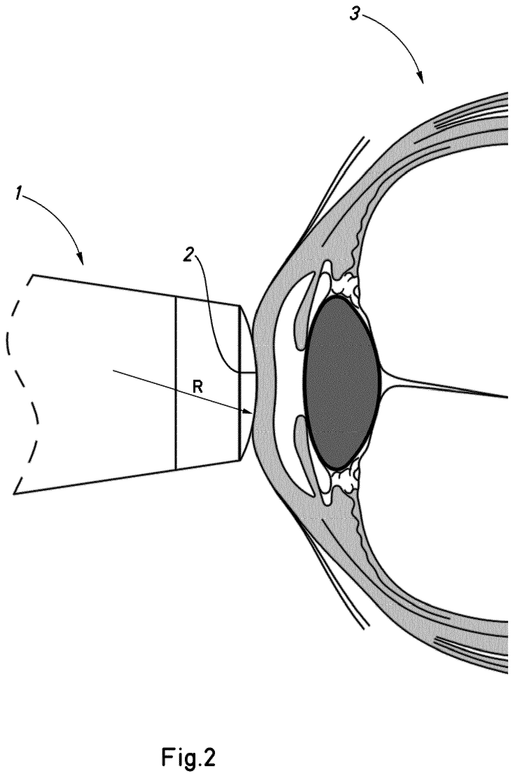

The surface of the convex curved apex is rigid to cause deformation of the cornea, as seen in Figure 2.

The present invention also discloses the use of an applanation tonometer or an optical bi-prism for measuring intraocular pressure, particularly in patients who have undergone LASIK or PRK refractive surgery.

For its better understanding, drawings of an embodiment of the new tonometer are attached. Such drawings are explanatory but not limitative examples.
Figure 1 shows a lateral view of the bi-prism with the curved apex applanation tonometer of the present invention.
Figure 2 shows a cross-sectional view of the contact position between the bi-prism with the curved convex apex (the new applanation tonometer) and the anterior surface of the cornea.
Figure 3 shows a cross-sectional view of the contact position between the bi-prism with the flat apex (GT) and the anterior surface of the cornea according to the state of the art.
Figure 4 shows a perspective view of the applanation tonometer with the convex-curved apex bi-prism in the bracket of a standard slit lamp.

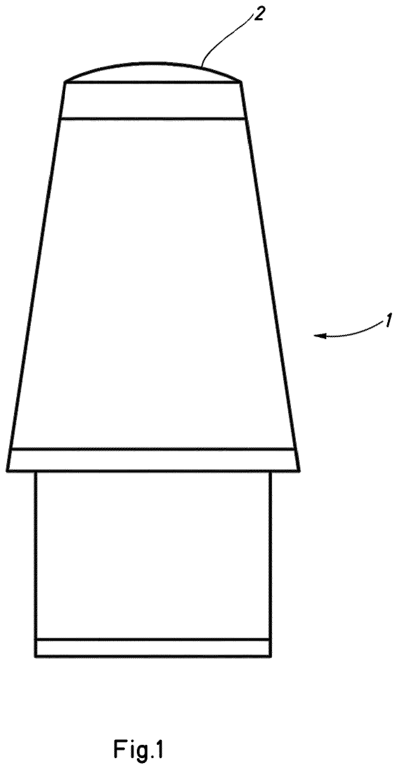

Figures 1 and 2 show a bi-prism convex-curved apex applanation tonometer according to the present invention.

The bi-prism -1- of Figure 1, has a convex curvature -2- at the apex, which will contact with the anterior surface of the cornea in order to measure IOP.

Figure 2 illustrates the contact position between the bi-prism -1- of the applanation tonometer with its convex curved apex -2-, with a mean surface radius -R- and the eye, when the IOP is being measured.

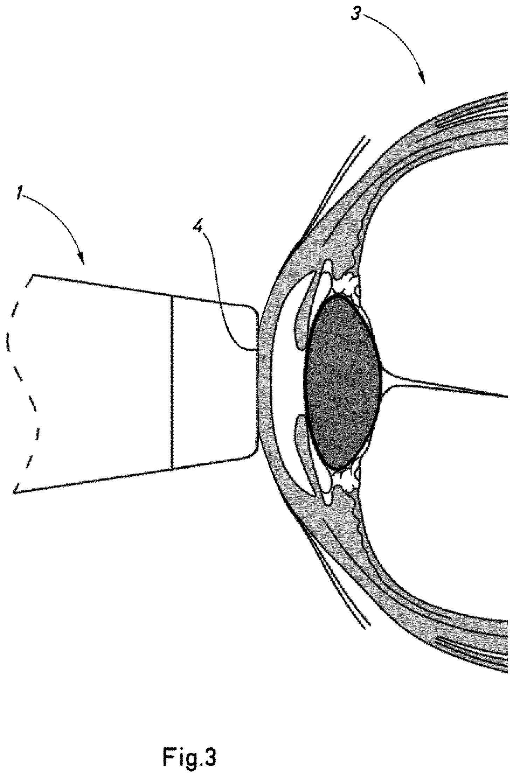

Figure 3 is included in a comparative way, and shows a cross-sectional view of the contact position between the bi-prism -1- of a GT with its flat surface -4- and the eye when the IOP is being measured.

In an experimental study performed in n=102 eyes of 102 patients operated on of LASIK or PRK, the lOPs were compared before and after 3 months of surgery measured with GT compared to two bi-prisms with curved convex apex with a surface radius curvature of 12.5 mm and 13.4 mm respectively. The results where satisfactory with a statistical power superior to 90% as the values obtained from IOP post surgery were very similar in the two bi-prisms of convex-curved apex compared to GT. In particular, preoperative IOP measured with GT correlated with a high statistical power in the postoperative period with the new convex curved apex tonometer. The bi-prism with the radius of curvature of 13.4 mm presented the best correlation.

While the invention has been presented and described with reference to embodiments thereof, it will be understood that these are not limitative of the invention, so that multiple construction or other details may be variable which may be apparent to those skilled in the art after to interpret the subject matter disclosed in the present disclosure, claims and drawings. Thus, all variants and equivalents will be included within the scope of the present invention if they fall within the scope of the following claims.

## Claims

1. An applanation tonometer for measuring IOP comprising an optical bi-prism with a distal apex intended to make contact with the anterior surface of the cornea, whose IOP is to be measured, **characterized in that** such optical bi-prism has a curved distal apex with a convex shape, seen from the outside of the bi-prism.

2. An applanation tonometer according to the preceding claim, **characterized by** an optical transparent bi-prism that has a convex curvature in its apex surface, with a mean radius in the range of 11.5 to 14 millimetres.

3. Optical bi-prism for applanation tonometer according to any of claims 1 to 2, **characterized by** a curved distal apex with a convex shape seen from the outside of the bi-prism.

4. Optical bi-prism according to claim 3, **characterized by** an apex of said bi-prism that has a curvature with a mean surface radius in a range of 11.5 to 14 millimetres.

5. Use of an applanation tonometer or an optical bi-prism according to anyone of the preceding claims, for measuring intraocular pressure, in particular who have undergone LASIK or PRK refractive surgery.

## Patentansprüche

1. Applanationstonometer zum Messen des Augeninnendrucks, umfassend ein optisches Doppelprisma mit einem distalen Apex, der dazu vorgesehen ist, mit der vorderen Fläche der Cornea in Kontakt zu kommen, deren Augeninnendruck gemessen werden soll, **dadurch gekennzeichnet, dass** ein solches optisches Doppelprisma einen gebogenen distalen Apex mit einer konvexen Form aufweist, betrachtet von der Außenseite des Doppelprismas.

2. Applanationstonometer nach dem vorhergehenden Anspruch, **gekennzeichnet durch** ein optisches transparentes Doppelprisma, das eine konvexe Krümmung in seiner Apexfläche mit einem mittleren Radius im Bereich von 11,5 bis 14 Millimeter aufweist.

3. Optisches Doppelprisma für ein Applanationstonometer nach einem der Ansprüche 1 bis 2, **gekennzeichnet durch** einen gekrümmten distalen Apex mit einer konvexen Form, betrachtet von der Außenseite des Doppelprismas..

4. Optisches Doppelprisma nach Anspruch 3, **gekennzeichnet durch** einen Apex des Doppelprismas, der eine Krümmung mit einem mittleren Oberflächenradius im Bereich von 11,5 bis 14 Millimeter aufweist.

5. Verwendung eines Applanationstonometers oder eines optischen Doppelprismas nach einem der vorhergehenden Ansprüche zum Messen des Augeninnendrucks, insbesondere nach einer LASIK oder PRK-Refraktionschirurgie.

## Revendications

1. Tonomètre à aplanation pour mesurer la PIO comprenant un biprisme optique avec une arête distale destinée à entrer en contact avec la surface antérieure de la cornée dont la PIO doit être mesurée, **caractérisé en ce qu'**un tel biprisme optique a une arête distale courbée avec une forme convexe, quand il est vu de l'extérieur du biprisme.

2. Tonomètre à aplanation selon la revendication précédente, **caractérisé par** un biprisme optique transparent qui a une courbure convexe dans sa surface d'arête, avec un rayon moyen dans la plage de 11,5 à 14 millimètres.

3. Biprisme optique pour tonomètre à aplanation selon l'une quelconque des revendications 1 ou 2, **caractérisé par** une arête distale courbée avec une forme convexe quand il est vu de l'extérieur du biprisme.

4. Biprisme optique selon la revendication 3, **caractérisé par** une arête dudit biprisme qui a une courbure avec un rayon de surface moyen dans une plage de 11,5 à 14 millimètres.

5. Utilisation d'un tonomètre à aplanation ou d'un biprisme optique selon l'une quelconque des revendications précédentes, pour mesurer la pression intraoculaire, en particulier après une chirurgie réfractive LASIK ou PKR.
